# EUROPEAN PATENT SPECIFICATION

(11) **EP 3 229 871 B1**
(45) Date of publication and mention of the grant of the patent: **15.03.2023**
(21) Application number: 15823400.5
(22) Date of filing: 08.10.2015
(51) Int. Cl.: A61M 5/28, A61M 5/142, A61M 5/44, G04B 37/12, G04B 47/00

(54) **WEARABLE INJECTION DEVICE**
TRAGBARE INJEKTIONSVORRICHTUNG
DISPOSITIF D'INJECTION PORTABLE

(30) Priority: 20.10.2014 NL 2013654
(43) Date of publication of application: 18.10.2017
(73) Proprietor: Witteman, Amber Helène Tamara, 2513 BX Den Haag (NL)
(72) Inventor: Witteman, Amber Helène Tamara, 2513 BX Den Haag (NL)
(74) Representative: Algemeen Octrooi- en Merkenbureau B.V.
(86) International application number: PCT/NL2015/050707
(87) International publication number: WO 2016/064266

(56) References cited:
- EP-A1- 1 700 615
- WO-A2-2009/015389
- WO-A2-2013/114221
- CN-A- 103 393 411
- DE-A1- 19 945 777
- DE-A1-102006 017 557
- DE-A1-102007 026 752
- FR-A1- 2 923 031
- FR-A1- 2 934 500
- US-A- 6 132 755

## Description

The present invention relates to a wearable injection device for injecting a medication through the skin of a body part.

### State of the art

From the state of the art such an injection device is known. This injection device is known as "EpiPen" and is used for the treatment of anaphylaxis, also known as anaphylactic shock. This is a suddenly occurring allergic response as a result of, for example, a food or insect allergy. Anaphylaxis can occur at any age. It is a relatively "new" condition that primarily affects children. It is life-threatening and requires immediate treatment. The number of people in need of such an EpiPen grows dramatically. In 2015, 22% of European children suffer from an aforementioned, serious allergy.

A disadvantage of this EpiPen is that it must not be forgotten by the person suffering from anaphylaxis, otherwise fatal consequences could be the result. Additionally, even if the aforementioned person is carrying the EpiPen, it is often the case that he or she is too late with administering the medication when anaphylaxis occurs. This because anaphylactic shock can lead to death in just tens of seconds and sometimes even seconds. Otherwise it should be noted that also with other diseases, such as diabetes or heart problems, quick administration is required.

CN 103 393 411 A discloses a watch-type injection device for injecting a general purpose substance. DE 10 2007 026752 A1 discloses a watch-type injection device with a refillable reservoir. DE 10 2006 017557 A1 discloses a watch-type device for carrying out measurements of glucose levels. FR 2 934 500 A1 discloses a wearable perfume bottle or oral spray bottle to be worn on the body with a wrist belt or the like.

### Aim of the invention

An aim of the invention is therefore to provide a wearable injection device that can not be forgotten by the person suffering from a disease requiring immediate administration of a medication, in particular anaphylaxis. Another aim of the invention is to provide a wearable injection device that can be operated in a very fast manner (by the person or by a bystander, such as a parent when the person concerns a child) to increase the chances of saving the abovementioned person's life, or to ameliorate emerging symptoms on the spot.

### Description of the invention

Thereto, according to the invention, a wearable injection device according to claim 1 is provided.

Due to the above injection device being attached to a body part, as a result of which the abovementioned wearable state is achieved, the person concerned always carries the injection device with him or her.

The injection side of the housing is provided with a closable opening, that is arranged for allowing the one or more injection members to move towards the skin through this opening in the injection state. With the help of such a closable opening, very rapid access is provided to the underlying skin, such that the chances of saving the person's life further increase, or the chances of severe symptoms occurring immediately decrease.

The housing is provided with an opening operation means that causes the closable opening to open upon operation thereof.

Additionally, when immediate intervention with the medication is required, the person or a bystander can operate the activation means as a result of which the medication is injected directly through the skin on the injection side.

The chances of the person's life being saved are thus increased dramatically. Additionally, the chances of the person ending up in hospital with severe consequences are dramatically decreased.

The wearable state by the way concerns the state wherein the injection device is carried by the person and wherein the injection device is releasably connected to the body part.

An embodiment concerns an aforementioned injection device, wherein the housing is provided with a lid means for providing access to the medication space. Thus, if desired, the person can easily replace the medication, for instance when the expiration date thereof has passed.

Another embodiment concerns an aforementioned injection device, wherein the housing comprises a user side, that is situated at a distance from the skin in the wearable state, wherein the lid means is provided at this user side. Thus, the person has even easier access to the medication space, wherein he or she can even better observe the condition of the medication in the medication space (or the condition of the medication space itself). Besides, the medication space thus is better accessible for cleaning and the like. Preferably, the medication space is also watertight.

Preferably, the housing therein is provided with a lid operation means that opens the lid means upon operation thereof. Such a lid operation means comes in handy when the medication in the form of a medication container with a casing is received in the medication space, wherein the activation means is arranged on the medication container. With a single action, access can thus be obtained to that activation means, while at the same time it is prevented that the activation means is activated by accident.

In particular, the body part concerns a limb.

An advantageous embodiment concerns, as stated before, an aforementioned injection device, wherein the medication is received in a casing of a medication container, wherein the medication space is arranged for receiving the medication container in the wearable state. Such a medication container with casing is quickly exchangeable and removable. Thus, the same injection device can be used with multiple types of medications, if desired.

An embodiment concerns an aforementioned injection device, wherein the medication container is provided with one or more injection members, and the activation means for activation of the injection members. Hygiene is thus facilitated, because the medication container is disposed of after use, together with the injection members, that from the perspective of hygiene should only be used once. Preferably, only injection systems are used having EMA-approval.

More preferably, the lid operation means and the opening operation means are formed by a single operation means, such that with a single action the medication space and the closable opening are opened and the device is immediately ready for injection.

In an advantageous embodiment, the closable opening comprises a diaphragm shutter, wherein upon activation of the opening activation means a diaphragm opening is created, through which the one or more injection members can move towards the skin.

A special embodiment concerns an aforementioned injection device, wherein the body part is the wrist of an arm and the skin is the skin of the wrist, wherein the attachment means is arranged for wearable attachment of the injection device to the wrist. By carrying the injection device around the wrist, accessibility thereof is further increased.

Therein, the injection device is preferably embodied as a wrist watch. Thus, the injection device can be carried around without drawing attention.

Preferably, the lid operation means or the opening operation means therein are embodied as a the control knob of a wrist watch, such that the injection device can be carried around without drawing attention, but is quickly ready for injection when required. Injection can even occur without the environment surrounding the person knowing injection has occurred, for instance when this person suffers from diabetes.

An embodiment relates to an injection device according to claim 11.

The medication container preferably comprises a pressure system that can be activated by the activation means, wherein, upon activation the medication is pushed through the one or more injection members by the pressure system. Thus, the medication can be injected into the body part, through the skin, very rapidly.

The medication concerns, as mentioned before, a medication that is intended for the treatment of conditions that require immediate treatment with the medication, such as anaphylaxis (epinephrine), heart conditions (adrenaline) or diabetes (insulin).

The medication container preferably comprises a self-cooling graphene paste for cooling the medication. Thus, medications can be kept cool even at high environmental temperatures, even up to 125°C.

Advantageously, the medication container can have the shape of a flattened pill or cylinder (flattened means: shortened along the centerline of the cylinder). The outer diameter of the medication container (i.e. the plane parallel to the skin), in particular the flattened pill or cylinder, preferably is 1-10 cm, such as 1, 2, 3, 4, 5, 6, 7, 8, 9, or 10 cm. Furthermore, the medication container preferably is suitable for only single use.

### Brief description of the drawings

The invention will be explained below with reference to an exemplary embodiment shown in the drawings. Therein:
Fig. 1 shows a perspective view of an embodiment of the injection device as a wrist watch, wherein the injection side is viewed;
Fig. 2 shows an exploded view of an embodiment of the housing with the medication container received therein;
Fig. 3 shows a view of the user side of the injection device, wherein the lid means is in an open state;
Fig. 4 shows a cross-section of an embodiment of the medication container, wherein the inside of the medication container is visible; and
Fig. 5 shows an exploded view of an embodiment of the housing with the medication container received therein, wherein the lid means is embodied as a screwcap.

### Detailed description of the drawings

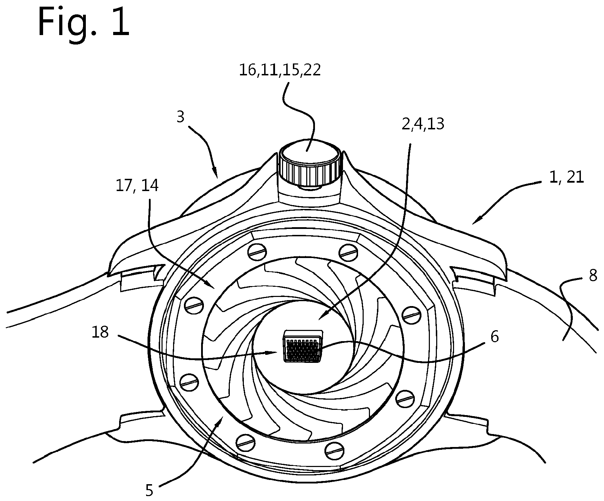

Figures 1-4 will be discussed in conjunction. Figure 1 shows a wearable injection device 1 in the form of a wrist watch 21 for wearable attachment to a body part, in particular a limb, such as a wrist of an arm, for injecting a medication 2 through the skin of the body part in a wearable state. The wrist watch 21 may concern a so-called "smartwatch", such as an Apple^{®} Watch. The injection device 1 as shown comprises a flat cylindrical housing 3, with a flattened cylindrical medication space 4 that comprises a medication 2 in the wearable state, an injection side 5 that is adjacent to the skin in the wearable state, wherein in the wearable state one or more injection members 6 are received in the housing 3 with an activation means 7 to activate the one or more injection members 6 for injecting the medication through the skin on the injection side, in an injection state. Furthermore, the injection device 1 comprises an attachment means 8, in the form of a wrist strap, that is connected to the housing 3 in the wearable state, releasably attaching the injection device 1 to the body part, and stably positioning the housing 3 with respect to the skin in the wearable state. The injection members 6 usually concern needles and preferably microneedles, although several types of injection members can be arranged in/on the ampoule. These microneedles preferably have a maximum length of 0.5 mm. The injection members 6 can be arranged on an injection member surface of approximately 0.5 x 0.5 cm, for instance with a longitudinal row of 2-8 injection members, more preferably around 4 injection members, and a transverse row having an equal amount of injection members 6. The injection device 1 is not thicker than 2, preferably 1 cm, at maximum (measured from the injection side 5 up to the point on the housing 3 that, seen in a direction perpendicular to the skin, is situated the furthest away from the injection side 5). This to improve wearability and invisibility. The outer diameter, i.e. in the direction parallel to the skin, is 5-10 cm at maximum, more preferably 7-8 cm.

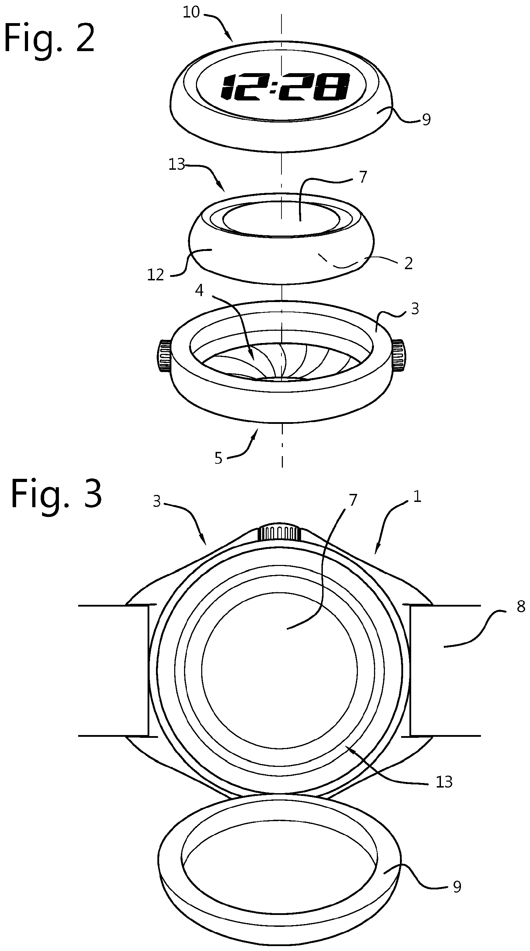

As shown in figures 2 and 3, the housing 3 of the injection device 1 can be provided with a lid means 9, such as a circular lid, for providing access to the medication space 4. This lid means 9 can advantageously comprise the screen of the wrist watch 21. The lid means 9 can be hingeably attached to the housing 3.

The housing 3 comprises a user side 10, i.e. the side comprising the screen, that is situated at a distance from the skin in the wearable state, wherein the lid means 9 is provided at this user side 10.

The housing 3 is provided with a lid operation means 11 that opens the lid means 9 upon operation thereof. Preferably, audible instructions are provided upon opening of the lid means 9, for instance from a speaker arranged in the housing 3. More preferably, the instructions are spoken and spoken/recorded with the help of the voice of someone the person knows, the person himself/herself, or somebody evoking positive feelings with the person to be treated, or in any case causes the person to follow the instructions. When a child is the person requiring immediate treatment, this may concern the voice of the child's mother. On the other hand the instructions can be used to instruct a bystander how the person to be treated needs to be injected with the help of the injection device.

An embodiment additionally provides the carrying out of a glucose measurement - before injection - when the person suffers from diabetes. Thus, the right dose of the medication (insulin) is administered, which is vital of course. This glucose measurement is preferably carried out by means of a light sensor arranged in the housing 3 at the skin side.

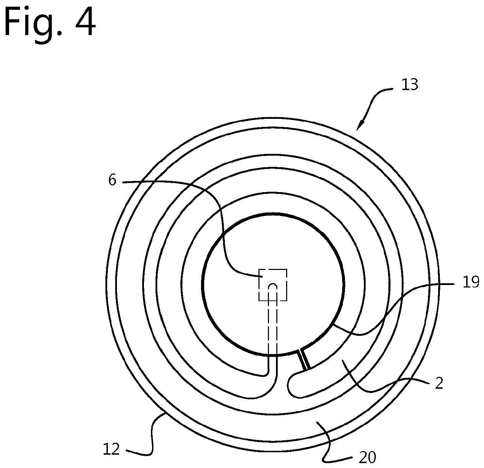

As shown, the medication 2 is received in a casing 12 of a removable medication container 13, wherein the medication space 4 is arranged for receiving this medication container 13 in the wearable state. Figure 4 shows a cross-section of this medication container 13. This medication container 13 is provided with one or more injection members 6, in the wearable state near the injection side, and the activation means 7 for activation of these injection members 6. The activation means 7 concerns a push button at that side of the medication container 13 that is situated near the user side 10 in the wearable state. Upon pressing the push button injection takes place. The medication container 13 can comprise multiple (adjustable) doses or multiple types of medications: when the patient suffers from multiple conditions, that could require immediate treatment, these conditions can be fought with one medication container 13.

The injection side 5 of the housing 3 in the form of a watch case is provided with a closable opening 14, that is arranged for allowing the one or more injection members 6 to move towards the skin through this opening 14. The housing 3 is furthermore provided with an opening operation means 15, embodied as a control knob 22 of the wrist watch 21, that upon operation thereof causes the closable opening 14 to open. The lid operation means 11 and the opening operation means 15 are formed by a single operation means 16, i.e. the same control knob 22. The control knob 22 preferably is embodied as a safety pin that can be pulled from the housing 3 to cause the lid means 9 and the opening 14 to open. The closable opening 14 comprises a diaphragm shutter 17. Upon operation of the opening operation means 15, in casu the control knob 22, a diaphragm opening 18 is created, through which the one or more injection members 6 can move towards the skin.

The cross-section of figure 4 shows that the medication container 13 in the form of an ampoule or a flattened pill comprises a pressure system 19, that can be activated by the activation means 7 in the form of a push button, wherein, upon activation, i.e. pushing on the push button, the medication 2 is pushed through the one or more injection members for injection into the body part. Figure 4 also shows that the medication container 13 comprises a self-cooling graphene paste 20 for cooling the medication 2. Preferably, the casing 12 is made of hard plastic, such as rubber, while at the position of the push button a soft plastic is used, such as soft rubber.

Advantageously, the injection device 1 can be provided with location determination means, such as a GPS chip, to be able to determine the position of the wearing person. Furthermore, an alarm system may be provided that automatically alarms the emergency services. Preferably, the position is immediately transmitted when the lid operation means 11 is operated. This position can be displayed, for instance on a smartphone of somebody close to the person or an acquaintance of the person to be treated, by means of a suitable app, such as Google Maps.

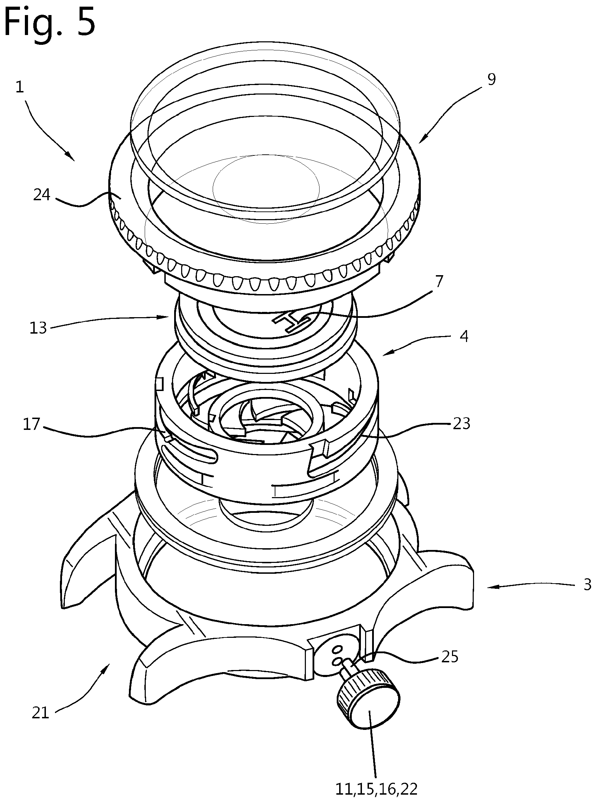

Figure 5 shows an exploded view of an embodiment of the housing 3 with the medication container 13 received therein, wherein the lid means 9 is embodied as a screw cap 24. When the control knob 22 (embodied as a safety pin) is operated, i.e. when the safety pin is pulled from the housing 3, blocking pins 25 arranged on the safety pin unblock the movement of the screwcap 24. The screwcap 24 is arranged with one or more radially inwardly extending guidance cams (not shown) in one or more corresponding guidance grooves 23. When the screwcap 24 is rotated clockwise (after removing the safety pin), the one or more guidance cams move through the one or more guidance grooves 23, wherein the diaphragm shutter 17 is gradually opened. At the end of the movement, the screwcap 24 can be removed from the one or more guidance grooves 23 substantially vertically. Subsequently, the medication container 13 can be pressed from above to inject the medication through the diaphragm shutter 17 that has been opened in the meantime.

Furthermore, it is conceivable that a second diaphragm shutter or a similar cap/lid is provided is situated below the diaphragm shutter 17 for additional protection, which opens when the safety pin is removed.

It should be clear that the description above is intended to illustrate the operation of preferred embodiments of the invention, the scope of which is defined by the appended claims.

### List of reference numerals

- 1.: Wearable injection device
- 2.: Medication
- 3.: Housing
- 4.: Medication space
- 5.: Injection side
- 6.: Injection members
- 7.: Activation means
- 8.: Attachment means
- 9.: Lid means
- 10.: User side
- 11.: Lid operation means
- 12.: Casing
- 13.: Medication container
- 14.: Closable opening
- 15.: Opening operation means
- 16.: Single operation means
- 17.: Diaphragm shutter
- 18.: Diaphragm opening
- 19.: Pressure system
- 20.: Graphene paste
- 21.: Wrist watch
- 22.: Control knob of wrist watch
- 23.: Guidance groove
- 24.: Screwcap
- 25.: Blocking pin

## Claims

1. Wearable injection device (1) for wearable attachment to a body part, for injecting a medication (2) through the skin of the body part in a wearable state, wherein the medication concerns a medication for the treatment of conditions requiring immediate treatment with the medication, such as anaphylaxis or diabetes, the injection device (1) being not thicker than 2 cm, i.e. in a direction perpendicular to the skin, and an outer diameter, i.e. in a direction parallel to the skin of 7-8 cm at maximum, and comprising:
- a housing (3), with a medication space (4) comprising an exchangeable medication container for single use, comprising the medication in the wearable state and an injection side (5) that borders the skin in the wearable state, wherein, in the wearable state, one or more injection members (6) are received in the housing, with an activation means (7) arranged on the medication container to activate the one or more injection members for injecting the medication through the skin on the injection side, in an injection state,
- an attachment means (8), that is connected to the housing in the wearable state and releasably attaches the injection device to the body part, and stably positions the housing with respect to the skin in the injection state,
**characterized in that** the injection side (5) of the housing (3) is provided with a closable opening (14), that is arranged for allowing the one or more injection members (6) to move towards the skin through this opening (14) in the injection state, wherein the housing is provided with an opening operation means (15) that upon operation thereof by a user causes the closable opening to open.

2. Injection device (1) according to claim 1, wherein the housing (3) is provided with a lid means (9) for providing access to the medication space (4).

3. Injection device (1) according to claim 2, wherein the housing (3) comprises a user side (10), that is situated at a distance from the skin in the wearable state, wherein the lid means (9) is provided at this user side.

4. Injection device (1) according to any one of the preceding claims, wherein the housing (3) is provided with a lid operation means (11) that upon operation thereof causes the lid means (9) to open.

5. Injection device (1) according to any one of the preceding claims, wherein the body part is a limb.

6. Injection device (1) according to claim 4, wherein the lid operation means (11) and the opening operation means (15) are formed by a single operation means (16).

7. Injection device (1) according to any one of the preceding claims, wherein the closable opening comprises a diaphragm shutter (17), wherein upon operation of the opening operation means (15) a diaphragm opening (18) is created, through which the one or more injection members (6) can move towards the skin.

8. Injection device (1) according to any one of the preceding claims, wherein the body part is the wrist of an arm and the skin is the skin of the wrist, wherein the attachment means (8) is arranged for wearable attachment of the injection device (1) to the wrist.

9. Injection device (1) according to claim 8, wherein the injection device (1) is embodied as a wrist watch (21).

10. Injection device (1) according to claim 9, wherein the lid operation means (11) according to claim 4, or the opening operation means (15) according to any one of the preceding claims, is embodied as a control knob (22) of a wrist watch.

11. Injection device (1) according to any one of the preceding claims, comprising the medication container (13) suitable for single use, for exchangeable placement in the medication space (4), with a casing (12), in which the medication (2) is received, wherein the medication concerns the medication intended for the treatment of conditions requiring immediate treatment with the medication, such as anaphylaxis or diabetes, wherein the medication container is provided with the one or more injection members (6) and the activation means (7) for activation of these injection members, to administer the medication via the skin at the injection side (5) of the injection device in the injection state, wherein the one or more injection members are arranged for moving towards the skin through the closable opening (14) in the injection state, when the opening operation means (15) upon operation thereof causes the closable opening (14) to open.

12. Injection device (1) according to claim 11, wherein the medication container (13) comprises a pressure system (19) that can be activated by the activation means (7), wherein, upon activation, the medication is pushed through the one or more injection members (6) by the pressure system.

13. Injection device (1) according to any one of the claims 11-12, wherein the medication container (13) comprises a self-cooling graphene paste (20) for cooling the medication.

14. Injection device (1) according to any one of the claims 11-13, wherein the medication container (13) has the shape of a flattened pill or cylinder.

## Patentansprüche

1. Tragbare Injektionsvorrichtung (1) zum tragbaren Anbringen an einem Körperteil zum Injizieren eines Medikaments (2) durch die Haut des Körperteils in einem tragbaren Zustand, wobei das Medikament ein Medikament für die Behandlung von Erkrankungen betrifft, die sofortige Behandlung mit dem Medikament erfordern, wie Anaphylaxie oder Diabetes, wobei die Injektionsvorrichtung (1) nicht dicker als 2 cm ist, d. h. in einer senkrecht zu der Haut verlaufenden Richtung, und einen Außendurchmesser, d. h. in einer parallel zu der Haut verlaufenden Richtung, von maximal 7 - 8 cm hat und Folgendes umfasst:
- ein Gehäuse (3) mit einem Medikamentraum (4), der einen austauschbaren Einweg-Medikamentbehälter umfasst, der das Medikament in dem tragbaren Zustand umfasst, sowie eine Injektionsseite (5), die in dem tragbaren Zustand an die Haut angrenzt, wobei in dem tragbaren Zustand ein oder mehrere Injektionsglieder (6) in dem Gehäuse aufgenommen sind, wobei ein Aktivierungsmittel (7) an dem Medikamentbehälter angeordnet ist, um das eine oder die mehreren Injektionsglieder zum Injizieren des Medikaments durch die Haut an der Injektionsseite in einem Injektionszustand zu aktivieren,
- ein Anbringmittel (8), das in dem tragbaren Zustand mit dem Gehäuse verbunden ist und die Injektionsvorrichtung freigebbar an dem Körperteil anbringt und das Gehäuse stabil bezüglich der Haut in dem Injektionszustand positioniert,
**dadurch gekennzeichnet, dass** die Injektionsseite (5) des Gehäuses (3) mit einer verschließbaren Öffnung (14) versehen ist, die dazu angeordnet ist, dass sich das eine oder die mehreren Injektionsglieder (6) in dem Injektionszustand durch diese Öffnung (14) zu der Haut hin bewegen können, wobei das Gehäuse mit einem Öffnungsbetätigungsmittel (15) versehen ist, das durch dessen Betätigung durch einen Benutzer das Öffnen der verschließbaren Öffnung veranlasst.

2. Injektionsvorrichtung (1) nach Anspruch 1, wobei das Gehäuse (3) mit einem Deckelmittel (9) versehen ist, um für Zugang zu dem Medikamentraum (4) zu sorgen.

3. Injektionsvorrichtung (1) nach Anspruch 2, wobei das Gehäuse (3) eine Benutzerseite (10) umfasst, die in dem tragbaren Zustand mit einem Abstand zu der Haut angeordnet ist, wobei das Deckelmittel (9) an dieser Benutzerseite vorgesehen ist.

4. Injektionsvorrichtung (1) nach einem der vorhergehenden Ansprüche, wobei das Gehäuse (3) mit einem Deckelbetätigungsmittel (11) versehen ist, das bei seiner Betätigung das Öffnen des Deckelmittels (9) veranlasst.

5. Injektionsvorrichtung (1) nach einem der vorhergehenden Ansprüche, wobei der Körperteil eine Gliedmaße ist.

6. Injektionsvorrichtung (1) nach Anspruch 4, wobei das Deckelbetätigungsmittel (11) und das Öffnungsbetätigungsmittel (15) durch ein einziges Betätigungsmittel (16) gebildet sind.

7. Injektionsvorrichtung (1) nach einem der vorhergehenden Ansprüche, wobei die verschließbare Öffnung einen Membranverschluss (17) umfasst, wobei bei Betätigung des Öffnungsbetätigungsmittels (15) eine Membranöffnung (18) erzeugt wird, durch die sich das eine oder die mehreren Injektionsglieder (6) zu der Haut hin bewegen können.

8. Injektionsvorrichtung (1) nach einem der vorhergehenden Ansprüche, wobei der Körperteil das Handgelenk eines Arms ist und die Haut die Haut des Handgelenks ist, wobei das Anbringmittel (8) zum tragbaren Anbringen der Injektionsvorrichtung (1) an dem Handgelenk angeordnet ist.

9. Injektionsvorrichtung (1) nach Anspruch 8, wobei die Injektionsvorrichtung (1) als eine Armbanduhr (21) ausgeführt ist.

10. Injektionsvorrichtung (1) nach Anspruch 9, wobei das Deckelbetätigungsmittel (11) nach Anspruch 4 oder das Öffnungsbetätigungsmittel (15) nach einem der vorhergehenden Ansprüche als ein Steuerknopf (22) einer Armbanduhr ausgeführt ist.

11. Injektionsvorrichtung (1) nach einem der vorhergehenden Ansprüche, umfassend den zur einmaligen Verwendung geeigneten Medikamentbehälter (13) für das austauschbare Platzieren in dem Medikamentraum (4) mit einer Aufnahme (12), in der das Medikament (2) aufgenommen ist, wobei das Medikament das Medikament betrifft, das für die Behandlung von Erkrankungen, die sofortige Behandlung mit dem Medikament erfordern, wie Anaphylaxie oder Diabetes, bestimmt ist, wobei der Medikamentbehälter mit einem oder mehreren Injektionsgliedern (6) und dem Aktivierungsmittel (7) zur Aktivierung dieser Injektionsglieder versehen ist, um das Medikament über die Haut an der Injektionsseite (5) der Injektionsvorrichtung in dem Injektionszustand zu verabreichen, wobei das eine oder die mehreren Injektionsglieder dazu angeordnet sind, sich in dem Injektionszustand durch die verschließbare Öffnung (14) zu der Haut hin zu bewegen, wenn das Öffnungsbetätigungsmittel (15) bei dessen Betätigung das Öffnen der verschließbaren Öffnung (14) veranlasst.

12. Injektionsvorrichtung (1) nach Anspruch 11, wobei der Medikamentbehälter (13) ein Drucksystem (19) umfasst, das durch das Aktivierungsmittel (7) aktiviert werden kann, wobei das Medikament mittels des Drucksystems durch das eine oder die mehreren Injektionsglieder (6) gedrückt wird.

13. Injektionsvorrichtung (1) nach einem der Ansprüche 11 - 12, wobei der Medikamentbehälter (13) eine selbstkühlende Graphen-Paste (20) zum Kühlen des Medikaments umfasst.

14. Injektionsvorrichtung (1) nach einem der Ansprüche 11 - 13, wobei der Medikamentbehälter (13) die Form einer abgeflachten Pille oder eines abgeflachten Zylinders hat.

## Revendications

1. Dispositif d'injection portable (1) pour une fixation portable à une partie du corps, pour injecter un médicament (2) à travers la peau de la partie du corps dans un état portable, dans lequel le médicament concerne un médicament pour le traitement d'affections nécessitant un traitement immédiat avec le médicament, telles que l'anaphylaxie ou le diabète, le dispositif d'injection (1) n'ayant pas une épaisseur supérieure à 2 cm, c'est-à-dire dans une direction perpendiculaire à la peau, et ayant un diamètre extérieur, c'est-à-dire dans une direction parallèle à la peau de 7 à 8 cm au maximum, et comprenant :
- un logement (3), avec un espace pour médicament (4) comprenant un récipient de médicament échangeable à usage unique, comprenant le médicament dans l'état portable et un côté injection (5) qui borde la peau dans l'état portable, dans lequel, dans l'état portable, un ou plusieurs éléments d'injection (6) sont reçus dans le logement, avec un moyen d'activation (7) agencé sur le récipient de médicament pour activer le ou les plusieurs éléments d'injection pour injecter le médicament à travers la peau sur le côté injection, dans un état d'injection,
- un moyen de fixation (8), qui est relié au logement dans l'état portable et fixe de manière amovible le dispositif d'injection à la partie du corps, et positionne de manière stable le logement par rapport à la peau dans l'état d'injection,
**caractérisé en ce que** le côté injection (5) du logement (3) est muni d'une ouverture refermable (14), qui est agencée pour permettre à ou aux plusieurs éléments d'injection (6) de se déplacer vers la peau à travers cette ouverture (14) dans l'état d'injection, dans lequel le logement est muni d'un moyen de manouvre d'ouverture (15) qui, lors de son actionnement par un utilisateur, provoque l'ouverture de l'ouverture refermable.

2. Dispositif d'injection (1) selon la revendication 1, dans lequel le logement (3) est muni d'un moyen formant couvercle (9) pour permettre l'accès à l'espace pour médicament (4).

3. Dispositif d'injection (1) selon la revendication 2, dans lequel le logement (3) comprend un côté utilisateur (10), qui est situé à une certaine distance de la peau dans l'état portable, dans lequel le moyen formant couvercle (9) est prévu sur ce côté utilisateur.

4. Dispositif d'injection (1) selon l'une quelconque des revendications précédentes, dans lequel le logement (3) est muni d'un moyen de manoeuvre de couvercle (11) qui, lors de son actionnement, provoque l'ouverture du moyen formant couvercle (9).

5. Dispositif d'injection (1) selon l'une quelconque des revendications précédentes, dans lequel la partie du corps est un membre.

6. Dispositif d'injection (1) selon la revendication 4, dans lequel le moyen de manoeuvre de couvercle (11) et le moyen de manoeuvre d'ouverture (15) sont formés par un seul moyen de manoeuvre (16).

7. Dispositif d'injection (1) selon l'une quelconque des revendications précédentes, dans lequel l'ouverture refermable comprend un obturateur à diaphragme (17), dans lequel lors de l'actionnement du moyen de manoeuvre d'ouverture (15), une ouverture de diaphragme (18) est créée, à travers laquelle le ou les plusieurs éléments d'injection (6) peuvent se déplacer vers la peau.

8. Dispositif d'injection (1) selon l'une quelconque des revendications précédentes, dans lequel la partie du corps est le poignet d'un bras et la peau est la peau du poignet, dans lequel le moyen de fixation (8) est agencé pour une fixation portable du dispositif d'injection (1) au poignet.

9. Dispositif d'injection (1) selon la revendication 8, dans lequel le dispositif d'injection (1) est conçu sous forme d'une montre-bracelet (21).

10. Dispositif d'injection (1) selon la revendication 9, dans lequel le moyen de manoeuvre de couvercle (11) selon la revendication 4, ou le moyen de manoeuvre d'ouverture (15) selon l'une quelconque des revendications précédentes, est conçu sous forme d'un bouton de commande (22) d'une montre-bracelet.

11. Dispositif d'injection (1) selon l'une quelconque des revendications précédentes, comprenant le récipient de médicament (13) adapté à un usage unique, pour être placé de manière échangeable dans l'espace pour médicament (4), avec un boîtier (12), dans lequel le médicament (2) est reçu, dans lequel le médicament concerne le médicament destiné au traitement des affections nécessitant un traitement immédiat avec le médicament, telles que l'anaphylaxie ou le diabète, dans lequel le récipient de médicament est muni du ou des plusieurs éléments d'injection (6) et du moyen d'activation (7) pour l'activation de ces éléments d'injection, pour administrer le médicament par la peau sur le côté injection (5) du dispositif d'injection dans l'état d'injection, dans lequel le ou les plusieurs éléments d'injection sont agencés pour se déplacer vers la peau à travers l'ouverture refermable (14) dans l'état d'injection, lorsque le moyen de manoeuvre d'ouverture (15), lors de son actionnement, provoque l'ouverture de l'ouverture refermable (14).

12. Dispositif d'injection (1) selon la revendication 11, dans lequel le récipient de médicament (13) comprend un système de pression (19) qui peut être activé par le moyen d'activation (7), dans lequel, lors de l'activation, le médicament est poussé à travers le ou les plusieurs éléments d'injection (6) par le système de pression.

13. Dispositif d'injection (1) selon l'une quelconque des revendications 11 et 12, dans lequel le récipient de médicament (13) comprend une pâte de graphène à refroidissement automatique (20) pour refroidir le médicament.

14. Dispositif d'injection (1) selon l'une quelconque des revendications 11 à 13, dans lequel le récipient de médicament (13) a la forme d'un cylindre ou d'une pilule aplati(e).
